# EUROPÄISCHE PATENTANMELDUNG

(11) **EP 1 698 285 A1**
(43) Veröffentlichungstag der Anmeldung: **06.09.2006**
(21) Anmeldenummer: 06003789.2
(22) Anmeldetag: 24.02.2006
(51) Int. Cl.: A61B 17/16, A61B 17/17, A61F 2/46

(54) **Medizinisches Instrument zum Einbringen von Mikrofrakturen in Knochen**

(30) Priorität: 03.03.2005 DE 102005010989
(71) Anmelder: Karl Storz GmbH & Co. KG, 78532 Tuttlingen (DE)
(72) Erfinder: Steinwachs, Matthias, Dr., 79112 Freiburg (DE); Berberich, Sascha, 78532 Tuttlingen (DE)
(74) Vertreter: Weller, Wolfgang

(57) **Zusammenfassung**

Ein medizinisches Instrument zum Einbringen von Mikrofrakturen in einem Knochen weist einen Körper (12) mit einer Spitze (22) an einem distalen Ende (14) auf. Das Instrument zeichnet sich dadurch aus, dass mehrere Spitzen (22) nach distal vorspringen.

## Beschreibung

Die Erfindung betrifft ein medizinisches Instrument zum Einbringen von Mikrofrakturen in Knochen, mit einem Körper, der an einem distalen Ende eine Spitze aufweist.

Das Einbringen von Mikrofrakturen in Knochen, das auch als "Microfracturing" bezeichnet wird, ist eine Technik zur Reparatur von Knorpeldefekten.

Instrumente zum Einbringen von Mikrofrakturen in Knochen, die manchmal auch als Microfraktur-Ahlen bezeichnet werden, sind bekannt und werden z.B. von der Firma Artrex GmbH, München, Deutschland unter der Bezeichnung "Chondro Pick" vertrieben.

Der Gelenkknorpel im Bereich des Kniegelenks des Menschen ist je nach Topographie unterschiedlich dick. Im Bereich der Patella kann er eine Schichtdicke von 7 bis 8 mm erreichen. Da der Gelenkknorpel keinen unmittelbaren Gefäß- oder Nervenanschluss besitzt, erfolgt seine Ernährung überwiegend durch Diffusion aus der Synovialflüssigkeit des Gelenkbinnenraums. Die Vernetzung unterschiedlicher Matrixkomponenten zur Knorpelgrundsubstanz ermöglicht eine mechanische Dämpfung und das fast reibungslose Gleiten der Gelenkflächen. Feingeweblich findet sich ein komplexer Aufbau aus Knorpelzellen (Chondrozyten), Collagenfasern und Proteoglykanen. Der gesunde Knorpel im Bereich des Knies eines erwachsenen Menschen ist in der Lage, Bewegungskräfte zu tolerieren, die ein Vielfaches des Körpergewichts betragen können.

Verletzungen des Gelenkknorpels stellen ein großes Problem im traumatologisch-orthopädischen Alltag dar. Das eingeschränkte Heilungsvermögen von Knorpel ist seit langem bekannt und ist im Wesentlichen in seiner besonderen Struktur und Anatomie begründet.

Ein Schaden an der Gelenkoberfläche, vor allen Dingen im Bereich der Belastungszonen der Gelenklauffläche, birgt daher das erhöhte Risiko einer weitgehenden Gelenkschädigung im Sinne einer frühzeitigen Arthrose.

Zur biologischen Rekonstruktion eines vollschichtigen Knorpelschadens bei kleinen und mittleren Defekten wird u.a. eine Technik eingesetzt, die als "Microfracturing" bezeichnet wird.

Hierbei werden mit Hilfe von spitzen Instrumenten kleine Öffnungen, sog. Mikrofrakturen, in den dem Knorpel unterliegenden Knochen eingestochen, die an das Knochenmark heranreichen. Durch diese Mikrofrakturen kann Blut und Knochenmark an die Oberfläche des Knochens gelangen.

Die Grundidee dieser Behandlungsmethode ist die Erzeugung eines Gerinnsels, das mit pluripotenten Stammzellen aus dem Knochenmark durchsetzt ist. Ein solches Gerinnsel wird manchmal auch als "Super Clot" bezeichnet. Die in diesem Gerinnsel enthaltenen Stammzellen können sich im Laufe des Heilungsprozesses zu Knorpelzellen differenzieren und neues Knorpelgewebe bilden.

Die zur Zeit eingesetzten Instrumente haben allerdings den Nachteil, dass jede Mikrofraktur im Knochen einzeln gesetzt werden muss. Dies ist ein sehr arbeitsaufwendiger Prozess.

Diese Technik hat ferner den Nachteil, dass es für einen Operateur schwierig ist, in den oft schwer zugänglichen Gelenkräumen eine gleichmäßige Verteilung der Mikrofraktur und daraus resultierend eine gleichmäßige Verteilung der Stammzellen und der Knorpelregeneration zu erreichen. Hierbei ist besonders darauf zu achten, dass die Mikrofrakturen nicht zu nahe aneinander heranreichen, da ansonsten zwischen ihnen Durchbrüche vorkommen können, die die Integrität des Knochens gefährden.

Es ist also Aufgabe der Erfindung, ein Werkzeug zu schaffen, mit dem das Microfracturing deutlich einfacher durchzuführen ist.

Erfindungsgemäß wird die Aufgabe durch ein medizinisches Instrument gelöst, von dessen distalem Ende mehrere Spitzen nach distal vorspringen.

Somit wird ein Werkzeug bereitgestellt, mit dem in einem einzigen Vorgang mehrere Mikrofrakturen geschaffen werden können.

Die Mikrofrakturen werden dabei in einer durch die Anordnung der Spitzen genau definierten Geometrie geschaffen. Damit können Durchbrüche zwischen den einzelnen geschaffenen Mikrofrakturen effektiv vermieden werden.

Mittels eines solchen Werkzeugs kann also das Microfracturing in einem einzigen Schritt erreicht werden. Durch die Vermeidung von Durchbrüchen zwischen den geschaffenen Mikrofrakturen wird außerdem die Integrität des zu behandelnden Knochens sichergestellt.

In einer Ausgestaltung der Erfindung sind die Spitzen gleichmäßig über das distale Ende verteilt.

Durch diese Maßnahme werden bei der Verwendung des medizinischen Instruments die Mikrofrakturen im Knochen in gleichmäßigen Abständen geschaffen. Dadurch kommt es zu einer gleichmäßigeren Verteilung des aus den Mikrofrakturen austretenden Bluts und Knochenmarks und damit der darin enthaltenen Stammzellen. Eine gleichmäßigere Verteilung der Stammzellen führt wiederum zu einer gleichmäßigeren Regeneration von Knorpelgewebe und somit zu einer verbesserten Heilung des Knorpeldefekts.

In einer weiteren Ausgestaltung der Erfindung springen die Spitzen von einer Endfläche des Instruments vor, wobei die Spitzen insbesondere eine Länge von 3 bis 5 mm aufweisen.

Durch die Bereitstellung einer Endfläche kann auf einfache Weise ein Anschlag geschaffen werden, durch den einem Operateur angezeigt wird, dass das Instrument zu einer ausreichenden Tiefe in den Knochen eingedrungen ist.

Durchschnittlich werden beim Microfracturing dabei Mikrofrakturen mit einer Tiefe von etwa 4 mm benötigt, um ein Austreten von Blut und Knochenmark zu erreichen. Durch die Kombination einer Endfläche, die als Anschlag dient, und Spitzen mit einer Länge von 3 bis 5 mm wird vermieden, dass die Spitzen zu tief in den Knochen eindringen und ggf. das Knochenmark verletzen.

In einer weiteren Ausgestaltung der Erfindung sind die Spitzen gehärtet.

Bei Knochen handelt es sich um ein relativ hartes Material, so dass es bei der mehrfachen Verwendung eines solchen medizinischen Instruments zu Abnutzungserscheinungen kommen kann. Ein Härten der Spitzen reduziert diese Abnutzungserscheinungen, so dass das Instrument länger effektiv einsetzbar ist. Da die Spitzen außerdem relativ klein und dünn sind, kann dadurch ein Abbrechen einer Spitze wirksam verhindert werden, so dass keine Fremdkörper im Knochen verbleiben.

In einer weiteren Ausgestaltung der Erfindung weist das medizinische Instrument ferner eine Führungshülse auf.

Eine solche Führungshülse kann von einem Operateur vor dem Einsetzen des eigentlichen Werkzeugs an der Operationsstelle positioniert werden und dient sozusagen als Zielgerät für das Einführen des eigentlichen Werkzeugs. Hierdurch wird ein besonders sicheres und gezieltes Einführen des Werkzeugs ermöglicht.

Durch die Verwendung einer solchen Führungshülse kann außerdem verhindert werden, dass der Knochen oder umliegendes Gewebe beim Einführen und Positionieren des Werkzeugs durch die distal vorspringenden Spitzen des Werkzeugs verletzt werden.

In einer weiteren Ausgestaltung der zuvor genannten Maßnahme weist die Führungshülse an einem distalen Ende eine Stanzklinge auf.

Bei der Reparatur von Knorpeldefekten ist es wichtig, dass sämtliche Stücke von defektem oder losem Knorpel vor der Durchführung des Microfracturing entfernt werden. Außerdem ist es für ein komplikationsfreies Regenerieren des Knorpels wichtig, dass die Ränder des gesunden Knorpels möglichst glatt präpariert sind.

Weist nun die Führungshülse an ihrem distalen Ende eine Stanzklinge auf, kann diese zusätzlich zu ihrer Aufgabe als Zielgerät für das Werkzeug noch als Knorpelstanze verwendet werden.

Eine solche Führungshülse kann arthroskopisch an einen Knorpeldefekt herangeführt werden und stanzt das Knorpelgewebe um den Defekt herum aus. Das so ausgestanzte Gewebe kann z.B. mit einem chirurgischen Löffel oder einer Kürette aus der Führungshülse entfernt werden. In einem nächsten Schritt kann dann das eigentliche Werkzeug eingeführt werden, um das Microfracturing durchzuführen.

Durch diese Kombination kann ein medizinisches Instrument geschaffen werden, mit dem auf einfache und schnelle Weise ein Knorpeldefekt zu reparieren ist.

In einer weiteren Ausgestaltung der Erfindung liegt das Instrument in verschiedenen Ausführungsformen bezüglich Form, Größe sowie Anzahl, Anordnung und Länge der Spitzen vor.

Durch diese Maßnahme kann ein Satz an Instrumenten geschaffen werden, aus dem ein Operateur sich das jeweils für den vorliegenden Defekt geeignete Instrument heraussuchen kann. Somit wird die Anwendbarkeit des Instruments deutlich erhöht.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend an Hand von ausgewählten Ausführungsbeispielen in Zusammenhang mit den beiliegenden Zeichnungen näher beschrieben und erläutert. Es zeigen:
- Fig. 1: eine perspektivische Ansicht eines medizinischen Instruments zum Einbringen von Mikrofrakturen in Knochen,
- Fig. 2: eine perspektivische Ansicht einer Führungshülse für das Instrument von Fig. 1,
- Fig. 3: stark schematisch einen ersten Schritt eines ersten Verfahrens zum Einbringen von Mikrofrakturen in Knochen im Schnitt,
- Fig. 4: stark schematisch einen zweiten Schritt des Verfahrens von Fig. 3,
- Fig. 5: stark schematisch einen dritten Schritt des Verfahrens von Fig. 3,
- Fig. 6: stark schematisch einen vierten Schritt des Verfahrens von Fig. 3,
- Fig. 7: stark schematisch einen fünften Schritt des Verfahrens von Fig. 3,
- Fig. 8: stark schematisch einen sechsten Schritt des Verfahrens von Fig. 3,
- Fig. 9: stark schematisch eine perspektivische Ansicht eines ersten Schritts eines zweiten Verfahrens zum Einbringen von Mikrofrakturen in Knochen,
- Fig. 10: stark schematisch eine perspektivische Ansicht eines zweiten Schritts des Verfahrens von Fig. 9,
- Fig. 11: stark schematisch eine perspektivische Ansicht eines dritten Schritts des Verfahrens von Fig. 9, und
- Fig. 12: stark schematisch eine perspektivische Ansicht eines vierten Schritts des Verfahrens von Fig. 9.

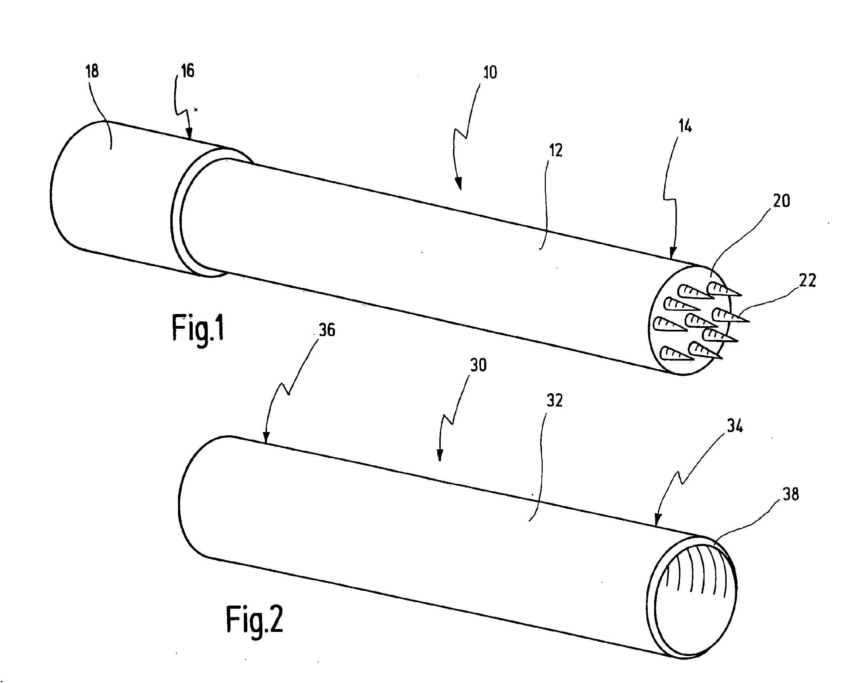

In Fig. 1 ist ein Instrument zum Einbringen von Mikrofrakturen in Knochen in seiner Gesamtheit mit der Bezugsziffer 10 bezeichnet.

Das Instrument 10 weist einen stabförmigen Körper 12 mit einem distalen Ende 14 und einem proximalen Ende 16 auf. Der Querschnitt des stabförmigen Körpers 12 ist oval.

Da es sich bei Knorpeldefekten häufig um Risse, also um längliche Defekte handelt, ist ein länglicher Querschnitt bevorzugt. Ein ovalärer Querschnitt ist bevorzugt, da dieser keine Ecken aufweist, die umliegendes Gewebe verletzen könnten.

Am proximalen Ende 16 erweitert sich der stabförmige Körper 12 zu einem Griff 18, der gleichzeitig dem Operateur eine Auflagefläche bietet, um das Instrument 10 mit seiner Hand oder einem Hammer in einen Knochen einzutreiben.

Das distale Ende 14 des Körpers weist eine plane Endfläche 20 auf. Von dieser planen Endfläche 20 erstrecken sich Spitzen 22, die hier in Form von Kegelspitzen ausgebildet sind, in distaler Richtung. Bei dieser Ausführungsform liegen neun Spitzen 22 vor, es können aber auch mehr oder weniger Spitzen 22 vorliegen.

Die Spitzen 22 sind gleichmäßig über die Endfläche 20 verteilt. Somit werden mit dem Werkzeug 10 Mikrofrakturen gleichmäßig verteilt in einem Knochen geschaffen. Dies führt zu einer gleichmäßigeren Verteilung von austretenden Stammzellen und einer gleichmäßigeren Heilung eines Knorpeldefekts.

Die Spitzen 22 sind mit Titannitrid gehärtet, um sie abnutzungsbeständiger zu machen, und dienen dazu, in einen Knochen einzudringen, um Mikrofrakturen zum Austritt von Blut und Knochenmark zu schaffen.

Werden diese Spitzen 22 nun in einen Knochen eingetrieben, ist dieses so weit möglich, bis die Endfläche 20 an dem Knochen zu liegen kommt. Die Endfläche 20 bietet somit einen Anschlag, der ein zu tiefes Eindringen der Spitzen 22 in den Knochen verhindert.

In Fig. 2 ist eine Führungshülse für das Werkzeug 10 von Fig. 1 in ihrer Gesamtheit mit der Bezugsziffer 30 bezeichnet.

Die Führungshülse 30 weist einen rohrförmigen Körper 32 auf, dessen Innenquerschnitt der Querschnittskontur des Körpers 12 des Werkzeugs 10 von Fig. 1 entspricht.

Der rohrförmige Körper 32 weist ein distales Ende 34 und ein proximales Ende 36 auf. Das distale Ende 34 ist so angeschliffen, dass eine Stanzklinge 38 gebildet wird, die sich um den gesamten Umfang des rohrförmigen Körpers 32 erstreckt.

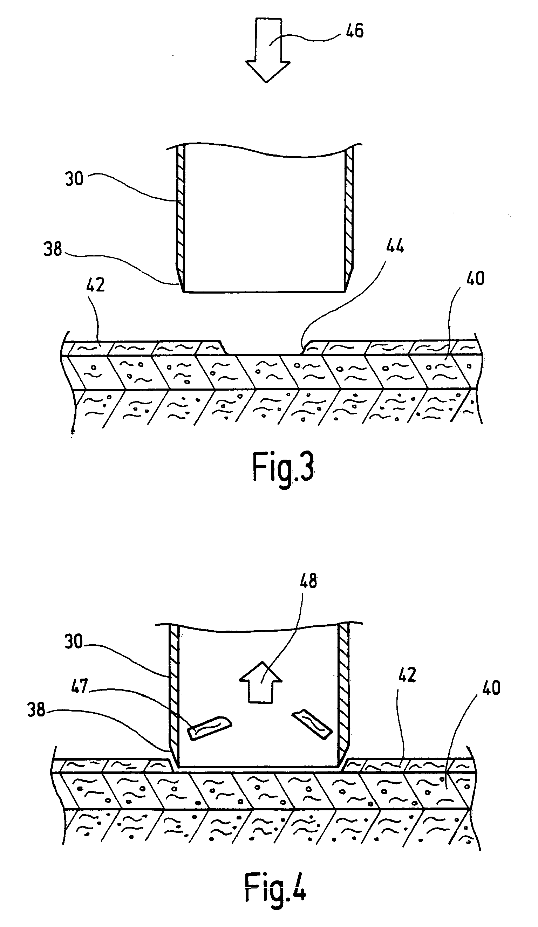

In Fig. 3 ist ein erster Schritt eines Verfahrens zum Einbringen von Mikrofrakturen in einen Knochen dargestellt. Hierbei wird unter arthroskopischer Sichtkontrolle die Führungshülse 30 an einen Knochen 40 im Kniegelenk herangeführt, der von einem Knorpel 42 bedeckt ist. Der Knorpel 42 weist dabei einen Knorpeldefekt 44 auf, der hier in Form eines Verlusts von Knorpelgewebe auftritt.

Der Knochen 40 ist hier mit einer ebenen Oberfläche dargestellt. Dies erfolgt nur aus Gründen der Einfachheit. Im Bereich des Kniegelenks werden fast ausschließlich gekrümmte Knochenoberflächen angetroffen.

Die zum Einführen der Führungshülse 30 verwendeten arthroskopischen Instrumente, wie z.B. ein Arthroskop zur Sichtkontrolle, sind hier nicht dargestellt.

Die Stanzklinge 38 der Führungshülse 30 ist um den Knorpeldefekt 44 herum ausgerichtet.

Die Hülse 30 wird nun in Richtung eines Pfeils 46 in den Knorpel 42 eingedrückt und auf den Knochen 40 zu bewegt, wobei die Stanzklinge 38 den Knorpeldefekt 44 ausstanzt.

Aus Fig. 4 wird ersichtlich, dass die Stanzklinge 38 der Führungshülse 30 nach der in Fig. 3 durch den Pfeil 46 angedeuteten Bewegung gegen den Knochen 40 zu liegen kommt. Sie hat hierbei Stücke 47 des Knorpels 42 ausgestanzt. Die von der Stanzklinge 38 ausgestanzten Stücke 47 des Knorpels 42 werden jetzt mit einem hier nicht dargestellten chirurgischen Löffel in Richtung eines Pfeils 48 aus dem Inneren der Führungshülse 30 entfernt. Somit ist von dem Knorpel 42 ein Bereich herausgetrennt, in dem die Oberfläche des Knochens 40 freiliegt.

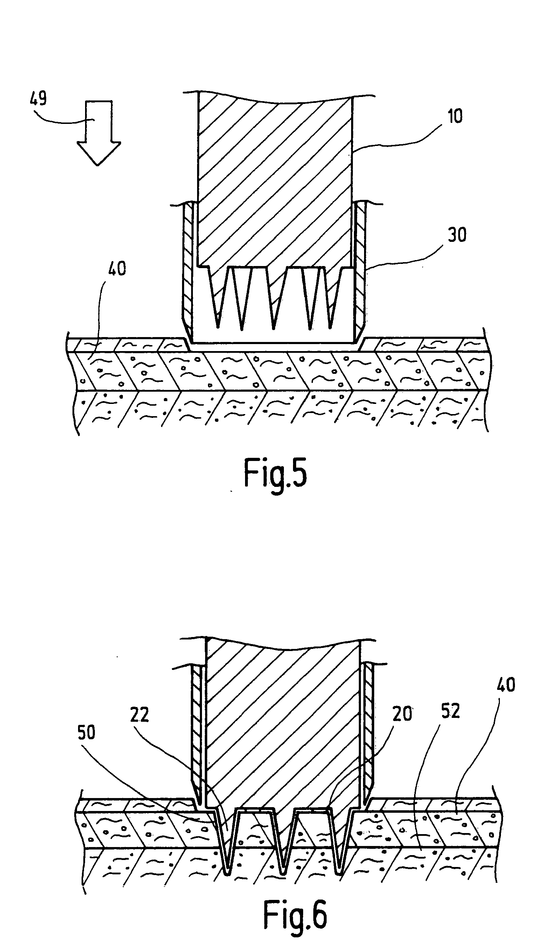

In Fig. 5 ist ein weiterer Schritt des Verfahrens dargestellt, wobei das Instrument 10 von proximal in die Führungshülse 30 eingeführt ist.

Das Instrument 10 wird nun in Richtung eines Pfeils 49 durch die Führungshülse 30 auf den Knochen 40 zu bewegt.

Wie aus Fig. 6 ersichtlich, dringen die Spitzen 22 in den Knochen 40 hinein, bis die Endfläche 20 gegen die Oberfläche des Knochens 40 zu liegen kommt. Dadurch werden in dem Knochen 40 Mikrofrakturen 50 geschaffen. Die Tiefe der Mikrofrakturen 50 wird durch das Anschlagen der Endfläche 20 gegen die Oberfläche des Knochens 40 begrenzt. Die Mikrofrakturen 50 reichen bis in ein dem Knochen 40 unterliegendes Knochenmark 52. Dieses Knochenmark 52 ist besonders reich an pluripotenten Stammzellen.

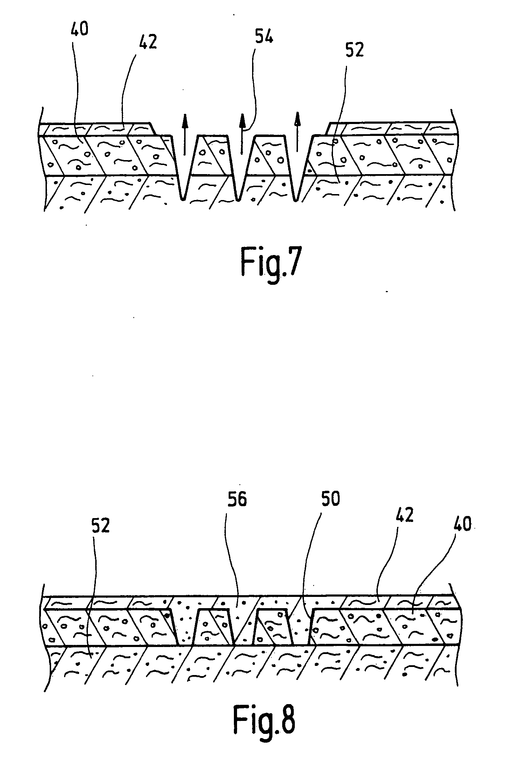

In Fig. 7 ist nun der Zustand des Knorpels 42, des Knochens 40 und des Knochenmarks 52 nach Entfernen der Führungshülse 30 und des Instruments 10 dargestellt.

Es wird hierbei ersichtlich, dass in dem Knochen 40 die Mikrofrakturen 50 verbleiben, die durch die Spitzen 22 des Instruments 10 gebildet wurden. Durch diese Mikrofrakturen 50 kann nun Blut, Stammzellen und anderes Material in Richtung der Pfeile 54 an die Oberfläche des Knochens 40 treten.

Dieses Material bildet an der Oberfläche des Knochens 40 ein mit pluripotenten Stammzellen durchsetztes Gerinnsel. Diese pluripotenten Stammzellen können sich im Laufe des Heilungsprozesses zu Knorpelzellen differenzieren und neues Knorpelgewebe bilden.

In Fig. 8 ist die Situation nach Abschluss des Heilungsprozesses dargestellt. Die aus dem Knochenmark 52 ausgetretenen Stammzellen haben sich differenziert und in den Mikrofrakturen 50 des Knochens 40 und den ausgeschnittenen Teilen des Knorpels 42 neue Knorpelzellen 56 gebildet. Hierdurch wurde der Knorpeldefekt vollständig behoben.

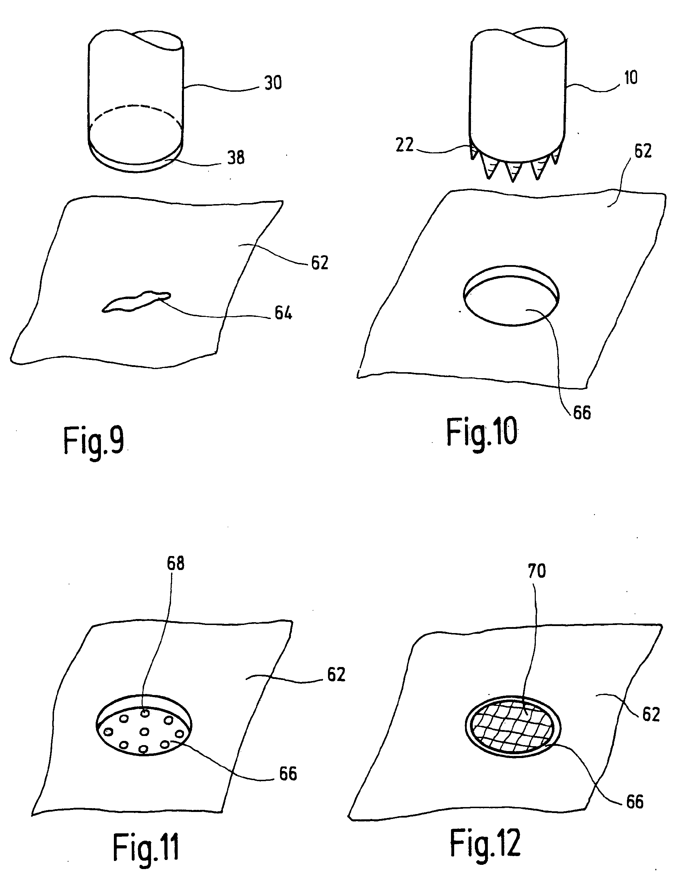

In Fig. 9 ist perspektivisch und stark vereinfacht ein zweites Verfahren zum Einbringen von Mikrofrakturen im Knochen dargestellt.

Perspektivisch ist ein Ausschnitt aus einem Knorpel 62 im Kniegelenk dargestellt, der einen Defekt 64 in Form eines Risses aufweist. Die Führungshülse 30 wird nun arthroskopisch so an den Defekt herangeführt, dass die Stanzklinge 38 um den Defekt 64 herum zu liegen kommt.

Die Stanzklinge 38 wird jetzt in den Knorpel 62 eingedrückt und das aus dem Knorpel 62 ausgestanzte Material wird nach dem Entnehmen der Führungshülse 30 mit einer Kürette entfernt.

Die Kürette, so wie andere bekannte arthroskopische Instrumente, wie z.B. ein Arthroskop zur Sichtkontrolle, die in diesem Verfahren verwendet werden, sind der Einfachheit halber hier nicht dargestellt.

In Fig. 10 ist die Situation nach Entfernen des Knorpelgewebes dargestellt.

In dem Knorpel 62 liegt jetzt ein ausgestanzter Bereich 66 vor, in dem ein unterliegender Knochen freiliegt. Es wird nun das Instrument 10 an den ausgestanzten Bereich herangeführt. Das Instrument 10 wird dann von dem Operateur in den ausgestanzten Bereich 66 eingedrückt und wieder daraus entfernt.

In Fig. 11 ist der Zustand nach dem Eindrücken und Entfernen des Instruments 10 von Fig. 10 dargestellt.

Es wird hierbei ersichtlich, dass in dem ausgestanzten Bereich 66 neun Mikrofrakturen 68 in einer definierten Geometrie gebildet wurden.

In einem nächsten Schritt wird nun, wie in Fig. 12 dargestellt, ein Vlies 70, das die gleiche Form und Größe wie der ausgestanzte Bereich 66 aufweist, in den ausgestanzten Bereich 66 des Knorpels 62 eingebracht. Die aus dem Knochenmark austretenden pluripotenten Stammzellen sammeln sich in diesem Vlies 70 an. Dieses Vlies 70 bietet den Zellen eine dreidimensionale Matrix, auf der diese wachsen können.

Das Vlies 70 selbst besteht aus Schweinecollagen und baut sich im Laufe des Heilungsprozesses ab. Somit wird ein besonders effektives und gezieltes Wachsen von neuem Knorpelgewebe sichergestellt.

## Patentansprüche

1. Medizinisches Instrument zum Einbringen von Mikrofrakturen in Knochen,
mit einem Körper (12), der an einem distalen Ende (14) eine Spitze (22) aufweist,
**dadurch gekennzeichnet, dass** mehrere Spitzen (22) nach distal vorspringen.

2. Medizinisches Instrument nach Anspruch 1, **dadurch gekennzeichnet, dass** die Spitzen (22) gleichmäßig über das distale Ende (14) verteilt sind.

3. Medizinisches Instrument nach Anspruch 1 oder 2, **dadurch gekennzeichnet, dass** die Spitzen (22) von einer Endfläche (20) des Instruments (10) vorspringen.

4. Medizinisches Instrument nach Anspruch nach Anspruch 3,
**dadurch gekennzeichnet, dass** die Spitzen (22) eine Länge von 3 bis 5 mm aufweisen.

5. Medizinisches Instrument nach einem der Ansprüche 1 bis 4,
**dadurch gekennzeichnet, dass** die Spitzen (22) gehärtet sind.

6. Medizinisches Instrument nach einem der Ansprüche 1 bis 5,
**dadurch gekennzeichnet, dass** es ferner eine Führungshülse (30) aufweist.

7. Medizinisches Instrument nach Anspruch 6, **dadurch gekennzeichnet, dass** die Führungshülse (30) an einem distalen Ende (34) eine Stanzklinge (38) aufweist.

8. Medizinisches Instrument nach einem der Ansprüche 1 bis 7,
**dadurch gekennzeichnet, dass** es in verschiedenen Ausführungsformen bezüglich Form, Größe sowie Anzahl, Anordnung und Länge der Spitzen (22) vorliegt.
